# EUROPEAN PATENT SPECIFICATION

(11) **EP 3 370 937 B1**
(45) Date of publication and mention of the grant of the patent: **20.07.2022**
(21) Application number: 16797483.1
(22) Date of filing: 07.11.2016
(51) Int. Cl.: B29C 45/14, B29C 45/16

(54) **MANUFACTURE OF MEDICAL DEVICES AND SUCH A MEDICAL DEVICES**
HERSTELLUNG EINES MEDIZINISCHES GERÄTES UND EIN SOLCHES MEDIZINISCHES GERÄT
PROCÉDÉ DE FABRICATION D'UN DISPOSITIF MÉDICAL ET UN TEL DISPOSITIF MÉDICAL

(30) Priority: 05.11.2015 GB 201519582
(43) Date of publication of application: 12.09.2018
(73) Proprietor: Intersurgical AG, 9490 Vaduz (LI)
(72) Inventor: MILLER, Andrew Neil, Wokingham Berkshire RG41 2RZ (GB); BOTTOM, David Simon, Wokingham Berkshire RG41 2RZ (GB)
(74) Representative: Adamson Jones
(86) International application number: PCT/EP2016/076860
(87) International publication number: WO 2017/077116

(56) References cited:
- WO-A1-93/10840
- JP-A- H01 174 426
- JP-A- H06 170 889
- US-A1- 2002 074 688
- US-A1- 2015 018 915
- US-A1- 2015 021 817
- US-A1- 2015 042 011

## Description

The present invention relates to tracheal intubation medical devices and a method of manufacturing the same. Tracheal intubation is the insertion of a plastic tube into the trachea in order to maintain an open airway in the patient. Intubation is often facilitated using a stylet or bougie as a guide for the intubation tube. A stylet is a malleable rod which may be disposed within the intubation tube to facilitate guiding of the tube. A bougie is a flexible introducer. The choice between using a stylet or a bougie as an intubation guide may depend on the situation and the speed of intubation required. As stylets are typically metallic rods, it is desirable to provide a covering on the rod, in order that the surface of the stylet is lubricious and biocompatible with the patient when inserted into the patient's trachea. An important consideration when manufacturing a stylet is achieving a plastic covering that is of even thickness. Known methods of covering the rod include inserting the rod into an extruded plastic sheath, dipping the rod into a molten plastic, spraying a molten plastic onto the rod, or compression moulding a coating onto the rod. These methods of manufacture may be costly and inefficient, and to date no method has been found to use a more preferable method such as injection moulding, whilst achieving a plastic overmoulding of the rod of generally even thickness and having smooth surface.

WO93/10840 discloses a protective cover for a blood-sample needle. An inner sheath surrounds the needle, and is housed within an outer sheath. The inner and outer sheaths are manufactured by two-component injection moulding. I' f

US 2015/ 018 915 discloses a stimulation lead comprising a series of electrodes, for use in medical applications.

US 2015/ 021 817 relates to segmented electrode leads for use in medical applications.

There has now been devised an improved method of manufacturing a medical device, which overcomes or substantially mitigate the aforementioned and/or other disadvantages associated with the prior art.

According to a first aspect of the invention there is provided a method of manufacturing a tracheal intubation medical device, comprising the following steps:
(a) providing at least one insert and a first sheath for the at least one insert, the first sheath including an inner sheath body and one or more sheath projections, wherein the at least one insert is malleable,
(b) providing a second injection moulding tool having a second mould chamber,
(c) locating the at least one insert and the first sheath within the second mould chamber, such that the at least one insert and the first sheath are supported within the second mould chamber by the one or more sheath projections, and
(d) injection moulding a second sheath about the inner sheath body and the at least one insert, between the one or more sheath projections. a method of manufacturing a medical device may comprise the following preceding steps:
   (a) providing a first injection moulding tool having a first mould chamber, the first mould chamber having one or more support projections extending inwardly into the chamber,
   (b) locating at least one insert within the first mould chamber, such that the at least one insert is supported by the one or more support projections, and
   (c) injection moulding a first sheath about the at least one insert, the first sheath including an inner sheath body and one or more sheath projections.

The method of manufacturing a medical device according to the invention is advantageous principally because the provision of the first sheath, including the one or more sheath projections, enables the at least one insert to be accurately positioned within the second mould chamber, and hence accurately positioned relative to the second sheath. This method of manufacture may therefore provide a more accurate arrangement for an insert and a sheath relative to prior art methods, and may also enable the sheath to have a substantially smooth exterior, with a reduced risk of the sheath catching on a patient's anatomy and causing trauma. In particular, although the one or more support projections of the first mould chamber may cause the first sheath to include openings, through which the at least one insert is exposed following injection moulding of the first sheath, the injection moulding of the second sheath may extend into and fill those openings.

In addition, the exterior surface of the second sheath may be substantially flush with the end surfaces of the one or more sheath projections. The medical device may therefore comprise at least one insert and a sheath, where the sheath consists of the first and second sheaths defined above, and the exterior surface of the sheath may be provided with a smooth exterior.

The at least one insert is malleable, and may be adapted substantially to retain its shape once deformed, eg at least against the action of gravity. The degree of malleability may be selected as required, eg for the particular function. The at least one insert may comprise a metal, eg aluminium or copper, or a plastics material, for example. The at least one insert may comprise a unitary element or a plurality of elements, such as a bundle of fibres. The at least one insert may be readily shaped by a user, eg using finger pressure only, and may substantially retain its shape once deformed. The material of the at least one insert may be relatively more rigid than the material(s) of the first and second sheaths. Where the medical device is adapted to be introduced into a patient, the at least one insert may be chosen to be sufficiently flexible to deform, eg lose its retained shape, as the device is removed from a patient.

The at least one insert may be elongate in form, and may have a substantially constant cross-section. The at least one insert may have the form of a rod, and may have any suitable cross-sectional shape. For example, the at least one insert may have a substantially circular or elliptical cross-sectional shape.

The medical device may be any device for tracheal intubation that requires at least one insert to be accurately positioned within a sheath. The present invention is particularly advantageous in relation to the manufacture of medical devices for tracheal intubation having a first portion that includes at least one insert, eg a malleable portion, and a second portion that does not include an insert, eg a flexible portion, as the use of injection moulding may enable an integral sheath to extend into both portions, thereby reducing the risk of kinks forming between the first and second portions. The present invention is particularly advantageous in relation to a medical device for tracheal intubation having a portion that includes at least one insert, eg a malleable portion, that is inserted into a patient, or utilised to insert another device into a patient. An example of a medical device for tracheal intubation having this arrangement is a device that may be used as either a stylet or a bougie.

The first mould chamber may define the inner sheath body and the one or more sheath projections of the first sheath. The one or more support projections of the first mould chamber may each have the form of a pin, and may have a contact surface that abuts the at least one insert when the insert is located within the first mould chamber. The first mould chamber may have a plurality of support projections, and the at least one insert may be retained or clamped between the support projections.

Where the at least one insert is elongate in form, eg in the form of a rod, the plurality of support projections may be disposed at a plurality of different longitudinal locations relative to the at least one insert, eg at substantially regular intervals along the length of the at least one insert. The plurality of projections may be arranged to substantially prevent bending of the at least one insert during injection moulding of the first sheath, eg caused by the flow of plastic under moulding pressure.

A set of support projections may be provided at positions along the at least one insert, with each set of projections retaining or clamping the at least one insert. Each set of support projections may comprise at least projections on each side of the at least one insert, and may comprise projections at substantially regularly spaced intervals, circumferentially of the at least one insert.

The one or more support projections may have a cross-sectional shape, eg an elliptical or rectangular shape, that has an increased dimension substantially in the direction of the longitudinal axis of the at least one insert. This may increase the contact area between the one or more support projections and the at least one insert. The one or more support projections may have a contact surface that mates with the exterior surface of the at least one insert. The contact surface of the one or more support projections may fit closely with the at least one insert.

The first mould chamber may have a plurality of feed locations, and may define a substantially uniform external cross-section for the inner sheath body, eg along substantially the length of the inner sheath body. The thickness of the inner sheath body will be selected dependent on the application, and also the need for the plastics material to flow about the at least one insert.

The inner sheath body of the first sheath may have substantially the same cross-sectional shape as the at least one insert, or may have a different cross-sectional shape. The inner sheath body may have a substantially uniform cross-section along the at least one insert. The inner sheath body may include openings, through which the at least one insert is exposed.

The first sheath and/or the inner sheath body of the first sheath may extend longitudinally along the entire length of the at least one insert, and may extend beyond one or more ends of the at least one insert. Alternatively, in order to provide a desired malleability and/or flexibility, eg at a particular location, the first sheath and/or the inner sheath body of the first sheath may extend longitudinally along a portion of the at least one insert, such that the at least one insert extends beyond an end, or both ends, of the first sheath and/or the inner sheath body of the first sheath, or an intermediate portion of the at least one insert extends between separated first sheaths.

The one or more sheath projections of the first sheath may extend outwardly from the inner sheath body. The one or more sheath projections may be upstanding from the external surface of the inner sheath body, and may project substantially perpendicularly relative to the longitudinal axis of the at least one insert. The one or more sheath projections may each include an end surface, which may face outwardly relative to the at least one insert, and which may be adapted to be disposed flush with the exterior surface of the second sheath formed by the second injection moulding. Alternatively, one or more sheath projections may be adapted to protrude beyond the exterior surface of the second sheath after the injection moulding surface to help prevent movement of the insert due to moulding pressure. Any protruding parts of the one or more sheath projections could then be removed prior to use of the device, for example by surface flaming, such that the end surfaces of the one or more sheath projections are substantially flush with the exterior surface of the second sheath in the final device.

The first sheath may have a plurality of sheath projections, and the at least one insert and the first sheath may be adapted to be supported on an inner surface of the second mould chamber by the sheath projections, such that the inner sheath body does not contact a surface of the second mould chamber.

Where the at least one insert is elongate in form, eg in the form of a rod, the plurality of sheath projections may be disposed at a plurality of different longitudinal locations relative to the at least one insert, eg at substantially regular intervals along the length of the at least one insert. The plurality of projections may be arranged to substantially prevent bending or movement of the at least one insert during injection moulding of the second sheath, eg caused by the flow of plastic under moulding pressure.

A set of sheath projections may be provided at positions along the at least one insert, with each set of projections being retained or clamped between inner surfaces of the second mould chamber. Each set of sheath projections may comprise at least projections on each side of the at least one insert, and may comprise projections at substantially regularly spaced intervals, circumferentially of the at least one insert.

The second mould chamber may have a plurality of feed locations, and may define a substantially uniform external cross-section for the second sheath, eg along substantially the length of the at least one insert. The thickness of the second sheath will be selected dependent on the application, and also the need for the plastics material to flow about the inner sheath body.

The second sheath may have substantially the same cross-sectional shape as the inner sheath body of the first sheath, or may have a different cross-sectional shape. The second sheath may have a substantially uniform cross-section along substantially the length of the insert and/or along substantially the length of the second sheath. The second sheath may extend about the one or more sheath projections, such that the one or more sheath projections are surrounded, save for end surfaces. The end surfaces of the one or more sheath projections may be substantially flush with the exterior surface of the second sheath. The exterior surface of the combined sheath, formed by the first and second sheaths, may be substantially uniform. The inner surfaces of the first and second mould tools may be polished.

The first and second sheaths may be formed of a plastics material, and may be formed from the same or substantially the same plastics material. Alternatively, the first and second sheaths may be formed of different plastics materials. The first and/or second sheaths may be formed of polyethylene, eg LDPE.

Since the end surfaces of the one or more sheath projections may be exposed, eg visible, and may be located at regular intervals along at least a portion of the device, the end surfaces of the one or more sheath projections may act as visual indicators, eg with respect to depth of insertion. For this application, the materials of the first and second sheaths may have different appearances, such as different materials with contrasting colours, textures or other visual features. The end surfaces of the one or more sheath projections may have the shape of visual indicia, eg markers, such as lines, or a series of individually identifiable indicia, such as a series of numerals or letters.

The first and second sheaths may surround the at least one insert, such that the patient is not exposed to a surface of the at least one insert.

The at least one insert may define a malleable portion of the device, which may be adapted to retain its shape once deformed, eg at least against the action of gravity. The device may also include an integral relatively flexible portion of the device, which is formed in the same injection moulding step as the second sheath. The second sheath may therefore extend beyond the at least one insert to define a relatively flexible portion of the device. A relatively flexible section may be provided at either end of a relatively malleable section containing at least one insert, eg containing a single insert. The cross-sectional shape and dimensions of the relatively flexible section may be substantially the same as the cross-sectional shape and dimensions of the malleable section, or may be different, depending on the application of the device.

The device is adapted for use as a guide or introducer for a tracheal tube. The device may comprise a stylet portion and a bougie portion. The device may hence be suitable for use as a bougie, as required, and for use as a stylet, as required.

Providing an elongate curved insert may also improve the accuracy of positioning and minimise or avoid movement with moulding pressure, by preventing or limiting longitudinal movement with material flow during the moulding process. Indeed, it may be possible to reduce the number of sheath projections on the first sheath while still ensuring and maintaining correct longitudinal positioning of an insert if a curved elongate insert is used.

According to a further aspect of the invention, there is provided a medical device for tracheal intubation manufactured by the method according to the invention. The device may comprise at least one insert surrounded by a first injection-moulded sheath, said first sheath comprising an inner sheath body and one or more sheath projections. The device may further comprise a second injection-moulded sheath that surrounds the inner sheath body, extending between the one or more sheath projections.

The construction of the medical device may be substantially as described above in relation to the method according to the invention.

An embodiment of the invention will be described in further detail below, by way of example only, with reference to the accompanying drawings, of which:
Figure 1 is a cross-section view of a device according to the invention;
Figure 2 is an enlarged cross-sectional view of a portion of the device;
Figure 3 is an enlarged side view of a portion of the device;
Figure 4 is a cross-sectional view of the device in an intermediate stage of manufacture; and
Figure 5 is a plan view of the device in an intermediate stage of manufacture.

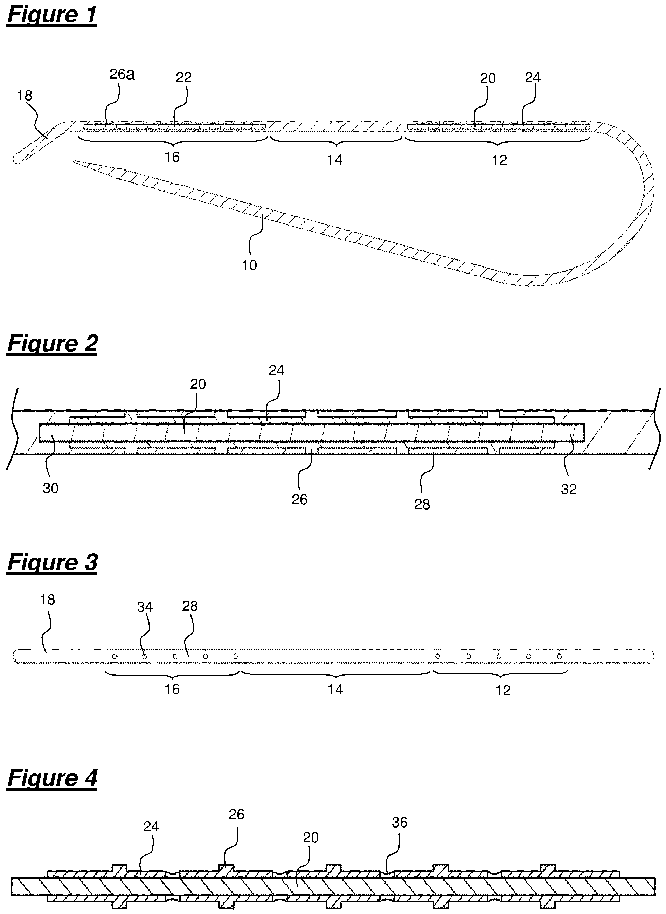

The device, as shown in Figure 1, comprises a flexible proximal end 10, a first malleable section 12, an intermediate flexible section 14, a second malleable section 16, and a flexible distal end 18.

The length of the device may be between 400 and 800 millimetres, eg approximately 650mm. The diameter of the apparatus may be between 3 and 10 millimetres.

The first malleable section 12 and the second malleable section 14 comprise metal rods 20, 22, which are surrounded by inner sheaths 24, 26a formed of a plastics material, eg LDPE. The inner sheaths 24, 26a are surrounded by a plastic material, in the form of an outer sheath, that makes up the flexible proximal end 10 and distal end 18, and the intermediate section 14.

The device can be used as both a bougie and a stylet. In a first configuration, the device is configured for use as a bougie, with the first malleable section 12 being bent to form a handle, and after insertion of the flexible distal end 18 into the trachea, the operator can railroad a tracheal tube over the flexible proximal end 10 and into the trachea. In a second configuration, the device is configured for use as a stylet where the second malleable section 14 is pre-formed and a tracheal tube is pre-loaded over the device. In this configuration, a handle may be formed by bending the flexible proximal end 10.

Figure 2 shows an enlarged view of the first malleable section 12. The rod 20 is a thin, elongate metal rod. The inner sheath 24 surrounds the rod 20. The rod 20 and inner sheath 24 are generally cylindrical, with the inner sheath 24 being concentric to the rod 20. The inner sheath 24 comprises projections 26 which extend from the surface of the inner sheath 24. The projections 26 are spaced along the length of the inner sheath and extend from regularly-spaced locations around the circumference of the inner sheath 24.

The outer sheath 28 surrounds the inner sheath 24 and the metal rod 20, such that the outer sheath 28 is concentric with the inner sheath 24 and the rod 20. The outer sheath 28 is made of the same material as the flexible portions of the device. The device is moulded such that the outer sheath 28 is unitary with the flexible portions of the device; the outer sheath 28 and flexible portions are moulded in a single injection moulding process. The inner sheath 24 does not cover the ends 30, 32 of the rod 20, however the ends 30, 32 of the rod 20 are surrounded by the outer sheath 28, which in this position is thicker, such that the surface of the device is smooth, and the diameter of the device is substantially uniform.

The outer sheath 28 surrounds the inner sheath 24, such that its exterior surface is flush with the end surfaces of the projections 26 of the inner sheath 24, and the projections 26 are thus exposed, as shown in Figure 3.

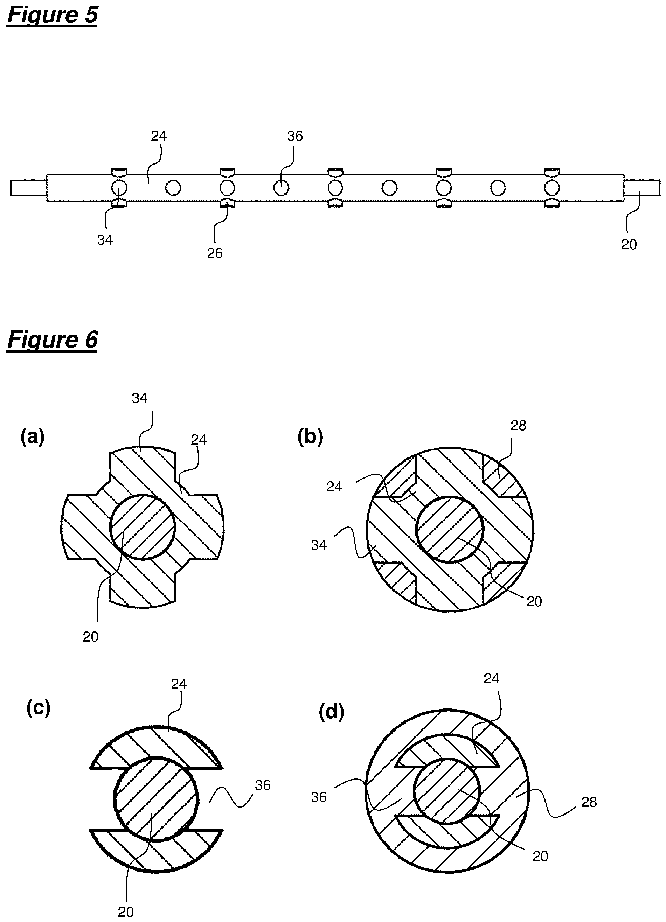

Figure 4 shows a cross-sectional view of a partial device during an intermediate stage of manufacture. The rod 20 and inner sheath 24 are shown, before the stage of manufacture in which the outer sheath 28 and flexible portions are moulded around the rod 20 and inner sheath 24. A top view of the partially constructed apparatus is shown in Figure 5.

As shown in Figures 4 and 5, the inner sheath 24 comprises openings 36, which are intermediately spaced between the projections 26. During manufacture of the inner sheath 24, the rod 20 is held in place by pins which contact the rod 20 in the position of the openings; the inner sheath 24 is created by injection moulding, and the plastic is injected into the mould such that it surrounds the rod 20 and the pins holding the rod 20 in place. The pins hold the rod 20 in place in the mould such that the inner sheath 24 is concentric to the rod 20.

During the stage of manufacture of the outer sheath 28 of the device, the outer sheath 28 is injection moulded around the inner sheath 24 such that the outer sheath 28 enters the openings 36 and surrounds the projections 26. The ends 34 of the projections 26 hold the rod 20 and inner sheath 24 in place in the mould, such that the thickness of the outer sheath 28, around the rod 20 and the inner sheath 24, is uniform along the length of the outer sheath 28and around the circumference of the outer sheath 28.

Figure 6(a) shows a cross-section though the rod 20 and the inner sheath 24, through a set of sheath projections 26, and Figure 6(b) shows the same cross-section following injection moulding of the outer sheath 28. The exterior surfaces of the outer sheath 28 and the sheath projections 26 are co-extensive, and flush with each other.

Figure 6(c) shows a cross-section though the rod 20 and the inner sheath 24, through a set of openings 36, and Figure 6(b) shows the same cross-section following injection moulding of the outer sheath 28. The outer sheath 28 extends into, and fills, the space that was previously defined by the openings 36.

## Claims

1. A method of manufacturing a tracheal intubation medical device, comprising the following steps:
(a) providing at least one insert 20 and a first sheath 24 for the at least one insert 20, the first sheath 24 including an inner sheath body and one or more sheath projections 26, wherein the at least one insert 20 is malleable,
(b) providing a second injection moulding tool having a second mould chamber,
(c) locating the at least one insert 20 and the first sheath 24 within the second mould chamber, such that the at least one insert 20 and the first sheath 24 are supported within the second mould chamber by the one or more sheath projections 26, and
(d) injection moulding a second sheath 28 about the inner sheath body and the at least one insert 20, between the one or more sheath projections 26.

2. The method as claimed in Claim 1, wherein the method comprises the following preceding steps:
(a) providing a first injection moulding tool having a first mould chamber, the first mould chamber having one or more support projections extending inwardly into the chamber,
(b) locating at least one insert 20 within the first mould chamber, such that the at least one insert 20 is supported by the one or more support projections, and
(c) injection moulding a first sheath 24 about the at least one insert 20, the first sheath including an inner sheath body and one or more sheath projections 26.

3. The method as claimed in Claim 1 or Claim 2, wherein the one or more support projections of the first mould chamber cause the first sheath to include openings 36, through which the at least one insert 20 is exposed following injection moulding of the first sheath 24, and the injection moulding of the second sheath 28 extends into and fills those openings 36.

4. The method as claimed in any preceding claim, wherein the at least one insert 20 comprises a unitary element or a plurality of elements, such as a bundle of fibres.

5. The method as claimed in any preceding claim, wherein the at least one insert 20 may be readily shaped by a user, eg using finger pressure only, and may retain its shape once deformed.

6. The method as claimed in any preceding claim, wherein the medical device has the form of a stylet or bougie, which is insertable through a tracheal tube.

7. The method as claimed in any preceding claim, wherein the first mould chamber has a plurality of support projections, and the at least one insert 20 is retained or clamped between the support projections.

8. The method as claimed in Claim 7, wherein the one or more support projections have a cross-sectional shape, eg an elliptical or rectangular shape, that has an increased dimension substantially in the direction of the longitudinal axis of the at least one insert 20.

9. The method as claimed in any preceding claim, wherein the one or more sheath projections 26 project substantially perpendicularly relative to the longitudinal axis of the at least one insert 20.

10. The method as claimed in any preceding claim, wherein a set of sheath projections 26 are provided at positions along the at least one insert 20, with each set of projections being retained or clamped between inner surfaces of the second mould chamber.

11. The method as claimed in any preceding claim, wherein the at least one insert 20 defines a malleable portion of the device, and the device also includes an integrally-formed, relatively flexible portion of the device, which is formed in the same injection moulding step as the second sheath 28.

12. The method as claimed in any preceding claim, wherein the second sheath 28 extends beyond the at least one insert 20 to define a relatively flexible portion of the device.

13. A tracheal intubation medical device manufactured by the method as claimed in any preceding claim.

14. The medical device as claimed in Claim 13, wherein the device comprises at least one insert 20 surrounded by a first injection-moulded sheath 24, said first sheath comprising an inner sheath body and one or more sheath projections 26.

15. The medical device as claimed in Claim 13 or Claim 14, wherein the device has the form of a bougie and/or stylet, and comprises at least a first malleable portion, longitudinally intermediate a flexible distal tip and a proximal flexible portion.

## Patentansprüche

1. Verfahren zur Herstellung eines medizinischen Gerätes zur trachealen Intubation, umfassend die folgenden Schritte:
(a) Bereitstellen mindestens eines Einsatzes 20 und einer ersten Hülle 24 für den mindestens einen Einsatz 20, wobei die erste Hülle 24 einen inneren Hüllenkörper und einen oder mehrere Hüllenvorsprünge 26 beinhaltet, wobei der mindestens eine Einsatz 20 verformbar ist,
(b) Bereitstellen eines zweiten Spritzgusswerkzeugs mit einer zweiten Formkammer,
(c) Anordnen des mindestens einen Einsatzes 20 und der ersten Hülle 24 innerhalb der zweiten Formkammer, so dass der mindestens eine Einsatz 20 und die erste Hülle 24 innerhalb der zweiten Formkammer von dem einen oder mehreren Hüllenvorsprüngen 26 abgestützt werden, und
(d) Spritzgießen einer zweiten Hülle 28 um den inneren Hüllenkörper und den mindestens einen Einsatz 20 zwischen dem einen oder mehreren Hüllenvorsprüngen 26.

2. Verfahren nach Anspruch 1, wobei das Verfahren die folgenden vorhergehenden Schritte umfasst:
(a) Bereitstellen eines ersten Spritzgusswerkzeugs mit einer ersten Formkammer, wobei die erste Formkammer einen oder mehrere Stützvorsprünge, die sich nach innen in die Kammer erstrecken, aufweist,
(b) Anordnen mindestens eines Einsatzes 20 innerhalb der ersten Formkammer, so dass der mindestens eine Einsatz 20 von dem einen oder mehreren Stützvorsprüngen abgestützt wird, und
(c) Spritzgießen einer ersten Hülle 24 um den mindestens einen Einsatz 20, wobei die erste Hülle einen inneren Hüllenkörper und einen oder mehrere Hüllenvorsprünge 26 beinhaltet.

3. Verfahren nach Anspruch 1 oder Anspruch 2, wobei der eine oder mehrere Stützvorsprünge der ersten Formkammer bewirken, dass die erste Hülle Öffnungen 36 beinhaltet, durch die der mindestens eine Einsatz 20 nach Spritzgießen der ersten Hülle 24 bloßgelegt wird, und das Spritzgießen der zweiten Hülle 28 sich in diese Öffnungen 36 erstreckt und diese füllt.

4. Verfahren nach einem vorhergehenden Anspruch, wobei der mindestens eine Einsatz 20 ein einheitliches Element oder eine Vielzahl von Elementen wie etwa ein Bündel von Fasern umfasst.

5. Verfahren nach einem vorhergehenden Anspruch, wobei der mindestens eine Einsatz 20 von einem Benutzer leicht geformt werden kann, z. B. nur unter Anwendung von Fingerdruck, und nach erfolgtem Verformen seine Form beibehalten kann.

6. Verfahren nach einem vorhergehenden Anspruch, wobei das medizinische Gerät die Form eines Mandrins oder einer Bougie, der/die durch einen Trachealtubus einführbar ist, aufweist.

7. Verfahren nach einem vorhergehenden Anspruch, wobei die erste Formkammer eine Vielzahl von Stützvorsprüngen aufweist und der mindestens eine Einsatz 20 zwischen den Stützvorsprüngen gehalten oder geklemmt wird.

8. Verfahren nach Anspruch 7, wobei der eine oder mehrere Stützvorsprünge eine Querschnittsform, z. B. eine elliptische oder rechteckige Form, aufweisen, die eine vergrößerte Abmessung im Wesentlichen in der Richtung der Längsachse des mindestens einen Einsatzes 20 aufweist.

9. Verfahren nach einem vorhergehenden Anspruch, wobei der eine oder mehrere Hüllenvorsprünge 26 im Wesentlichen senkrecht relativ zu der Längsachse des mindestens einen Einsatzes 20 hervorstehen.

10. Verfahren nach einem vorhergehenden Anspruch, wobei ein Satz von Hüllenvorsprüngen 26 an Positionen entlang des mindestens einen Einsatzes 20 bereitgestellt sind, wobei jeder Satz von Vorsprüngen zwischen Innenflächen der zweiten Formkammer gehalten oder geklemmt wird.

11. Verfahren nach einem vorhergehenden Anspruch, wobei der mindestens eine Einsatz 20 einen verformbaren Abschnitt des Gerätes definiert und das Gerät auch einen integral geformten, relativ flexiblen Abschnitt des Gerätes, der in demselben Spritzgussschritt wie die zweite Hülle 28 gebildet wird, beinhaltet.

12. Verfahren nach einem vorhergehenden Anspruch, wobei sich die zweite Hülle 28 über den mindestens einen Einsatz 20 hinaus erstreckt, um einen relativ flexiblen Abschnitt des Gerätes zu definieren.

13. Medizinisches Gerät zur trachealen Intubation, das durch das Verfahren nach einem vorhergehenden Anspruch hergestellt wird.

14. Medizinisches Gerät nach Anspruch 13, wobei das Gerät mindestens einen Einsatz 20 umfasst, der von einer ersten spritzgegossenen Hülle 24 umgeben ist, wobei die erste Hülle einen inneren Hüllenkörper und einen oder mehrere Hüllenvorsprünge 26 umfasst.

15. Medizinisches Gerät nach Anspruch 13 oder Anspruch 14, wobei das Gerät die Form einer Bougie und/oder eines Mandrins aufweist und mindestens einen ersten verformbaren Abschnitt, in Längsrichtung zwischen einer flexiblen distalen Spitze und einem proximalen flexiblen Abschnitt, umfasst.

## Revendications

1. Procédé de fabrication d'un dispositif médical d'intubation trachéale, comprenant les étapes suivantes :
(a) la fourniture d'au moins un insert 20 et d'une première gaine 24 pour ledit au moins un insert 20, la première gaine 24 comprenant un corps de gaine interne et une ou plusieurs saillies de gaine 26, ledit au moins un insert 20 étant malléable,
(b) la fourniture d'un second outil de moulage par injection possédant une seconde chambre de moule,
(c) le positionnement dudit au moins un insert 20 et de la première gaine 24 à l'intérieur de la seconde chambre de moule, de sorte que ledit au moins un insert 20 et la première gaine 24 soient supportés à l'intérieur de la seconde chambre de moule par lesdites une ou plusieurs saillies de gaine 26, et
(d) le moulage par injection d'une seconde gaine 28 autour du corps de gaine interne et dudit au moins un insert 20, entre lesdites une ou plusieurs saillies de gaine 26.

2. Procédé selon la revendication 1, ledit procédé comprenant les étapes précédentes suivantes :
(a) la fourniture d'un premier outil de moulage par injection possédant une première chambre de moule, la première chambre de moule comportant une ou plusieurs saillies de support s'étendant vers l'intérieur dans la chambre,
(b) le positionnement d'au moins un insert 20 à l'intérieur de la première chambre de moule, de sorte que ledit au moins un insert 20 soit supporté par lesdites une ou plusieurs saillies de support, et
(c) le moulage par injection d'une première gaine 24 autour dudit au moins un insert 20, la première gaine comprenant un corps de gaine interne et une ou plusieurs saillies de gaine 26.

3. Procédé selon la revendication 1 ou la revendication 2, lesdites une ou plusieurs saillies de support de la première chambre de moule amenant la première gaine à inclure des ouvertures 36, au travers desquelles ledit au moins un insert 20 est exposé suite au moulage par injection de la première gaine 24, et ledit moulage par injection de la seconde gaine 28 s'étendant dans ces ouvertures 36 et les remplissant.

4. Procédé selon l'une quelconque des revendications précédentes, ledit au moins un insert 20 comprenant un élément unitaire ou une pluralité d'éléments, tels qu'un faisceau de fibres.

5. Procédé selon l'une quelconque des revendications précédentes, ledit au moins un insert 20 pouvant être facilement mis en forme par un utilisateur, par exemple en utilisant uniquement la pression du doigt, et pouvant conserver sa forme une fois déformé.

6. Procédé selon l'une quelconque des revendications précédentes, ledit dispositif médical ayant la forme d'un stylet ou d'une bougie, qui peut être inséré à travers un tube trachéal.

7. Procédé selon l'une quelconque des revendications précédentes, ladite première chambre de moule comportant une pluralité de saillies de support, et ledit au moins un insert 20 étant retenu ou serré entre les saillies de support.

8. Procédé selon la revendication 7, lesdites une ou plusieurs saillies de support ayant une forme en coupe transversale, par exemple une forme elliptique ou rectangulaire, qui présente une dimension accrue sensiblement dans la direction de l'axe longitudinal dudit au moins un insert 20.

9. Procédé selon l'une quelconque des revendications précédentes, lesdites une ou plusieurs saillies de gaine 26 faisant saillie sensiblement perpendiculairement par rapport à l'axe longitudinal dudit au moins un insert 20.

10. Procédé selon l'une quelconque des revendications précédentes, une série de saillies de gaine 26 étant disposée dans des positions le long dudit au moins un insert 20, chaque série de saillies étant retenue ou serrée entre les surfaces internes de la seconde chambre de moule.

11. Procédé selon l'une quelconque des revendications précédentes, ledit au moins un insert 20 définissant une partie malléable du dispositif, et ledit dispositif comprenant également une partie relativement flexible du dispositif formée d'un seul tenant, qui est formée dans la même étape de moulage par injection que la seconde gaine 28.

12. Procédé selon l'une quelconque des revendications précédentes, ladite seconde gaine 28 s'étendant au-delà dudit au moins un insert 20 pour définir une partie relativement flexible du dispositif.

13. Dispositif médical d'intubation trachéale, fabriqué par le procédé selon l'une quelconque des revendications précédentes.

14. Dispositif médical selon la revendication 13, ledit dispositif comprenant au moins un insert 20 entouré d'une première gaine 24 moulée par injection, ladite première gaine comprenant un corps de gaine interne et une ou plusieurs saillies de gaine 26.

15. Dispositif médical selon la revendication 13 ou la revendication 14, ledit dispositif ayant la forme d'une bougie et/ou d'un stylet, et comprenant au moins une première partie malléable, longitudinalement intermédiaire entre une pointe distale flexible et une partie flexible proximale.
